# EUROPEAN PATENT SPECIFICATION

(11) **EP 3 968 978 B1**
(45) Date of publication and mention of the grant of the patent: **18.06.2025**
(21) Application number: 20724510.1
(22) Date of filing: 13.05.2020
(51) Int. Cl.: A61K 31/135, A61K 31/415, A61P 29/00

(54) **CO-CRYSTAL OF (RAC) TRAMADOL AND CELECOXIB FOR USE IN THE TREATMENT OF PAIN IN PATIENT WITH AN ADDICTION TO TRAMADOL**
CO-KRISTALL AUS (RAC) TRAMADOL UND CELECOXIB ZUR VERWENDUNG BEI DER SCHMERZBEHANDLUNG BEI PATIENTEN MIT EINER ABHÄNGIGKEIT VON TRAMADOL
CO-CRISTAL DE (RAC) TRAMADOL ET DE CÉLÉCOXIB DESTINÉ À ÊTRE UTILISÉ DANS LE TRAITEMENT DE LA DOULEUR CHEZ LES PATIENTS PRÉSENTANT UNE DÉPENDANCE AU TRAMADOL

(30) Priority: 14.05.2019 EP 19382377
(43) Date of publication of application: 23.03.2022
(73) Proprietor: ESTEVE PHARMACEUTICALS, S.A., 08038 Barcelona (ES)
(72) Inventor: ENCINA-GARCÍA, Gregorio-José, 08025 Barcelona (ES); PLATA-SALAMÁN, Carlos-Ramón, 08021 Barcelona (ES)
(74) Representative: Peters, Hajo
(86) International application number: PCT/EP2020/063250
(87) International publication number: WO 2020/229502

(56) References cited:
- EP-A1- 2 349 238
- EP-A2- 2 586 432
- WO-A1-2011/151080
- JP-A- 2017 190 328
- SEBASTIÁN VIDELA ET AL: "Single-dose pharmacokinetics of co-crystal of tramadol-celecoxib: Results of a four-way randomized open-label phase I clinical trial in healthy subjects", BRITISH JOURNAL OF CLINICAL PHARMACOLOGY., vol. 83, no. 12, 20 September 2017 (2017-09-20), GB, pages 2718 - 2728, XP055636240, ISSN: 0306-5251, DOI: 10.1111/bcp.13395
- HELENDOONER ET AL: "Pharmacokinetics ofTramadol andCelecoxib inJapanese andCaucasian Subjects Following Administration ofCo-Crystal ofTramadol-Celecoxib (CTC): ARandomised, Open-Label Study", EUROPEAN JOURNAL OF DRUG METABOLISM AND PHARMACOKINETICS, 28 June 2018 (2018-06-28), pages 63 - 75, XP055636279, Retrieved from the Internet <URL:https://www.ncbi.nlm.nih.gov/pmc/articles/PMC6394644/pdf/13318_2018_Article_491.pdf> [retrieved on 20191028]
- CARMEN ALMANSA ET AL: "): A Novel API-API Co-crystal for the Treatment of Pain", CRYSTAL GROWTH & DESIGN., vol. 17, no. 4, 20 March 2017 (2017-03-20), US, pages 1884 - 1892, XP055636403, ISSN: 1528-7483, DOI: 10.1021/acs.cgd.6b01848
- JOSÉ LÓPEZ-CEDRÚN ET AL: "Co-crystal of Tramadol-Celecoxib in Patients with Moderate to Severe Acute Post-surgical Oral Pain: A Dose-Finding, Randomised, Double-Blind, Placebo- and Active-Controlled, Multicentre, Phase II Trial", DRUGS IN R AND D, vol. 18, no. 2, 25 May 2018 (2018-05-25), pages 137 - 148, XP055636679, ISSN: 1174-5886, DOI: 10.1007/s40268-018-0235-y

## Description

The present invention relates to the use of co-crystals of tramadol and celecoxib for the treatment of pain, preventing the risk of an addiction to tramadol, thus, for the treatment of pain in a patient with an addiction to tramadol while reducing the abuse liability of tramadol.

### BACKGROUND

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis, or pain of unknown origin, such as fibromyalgia. Acute pain usually follows non-neural tissue injury, for example tissue damage from surgery or inflammation, or migraine.

Recently co-crystals of tramadol and celecoxib have been discovered that are used for the treatment of pain. Examples of such co-crystals are published under PCT in WO2011/044962 and are very effective in the treatment of pain, especially in the treatment acute and chronic medium to severe pain.

Tramadol is a synthetic, centrally acting analgesic agent with 2 distinct, synergistic mechanisms of action. It is both a weak opioid agonist with selectivity for the µ-receptor and a weak inhibitor of the reuptake of noradrenaline (norepinephrine) and serotonin (5-hydroxytryptamine; 5-HT). Its analgesic potency is claimed to be about one tenth that of morphine. Tramadol is used to treat both acute and chronic pain of moderate to (moderately) severe intensity. Tramadol is considered to be a relatively safe analgesic and is widely used.

The main adverse reactions to tramadol therapy are nausea, dizziness, and vomiting, particularly at the start of the therapy. At therapeutic doses, tramadol does not cause clinically relevant respiratory depression. Tramadol is contra-indicated, however, in patients with diminished respiratory function. Tramadol is generally considered as a medicinal drug with a low potential for dependence relative to morphine. Nevertheless, tramadol dependence has been shown to occur when used for prolonged periods of time (more than several weeks to months). Dependence to tramadol may occur when used within the recommended dose range but especially when used at supra-therapeutic doses. At supra-therapeutic doses and rarely at therapeutic doses, intoxications may occur. Symptoms of tramadol intoxication are similar to those of other opioid analgesics but may include serotonergic and noradrenergic components. Symptoms include central nervous system (CNS) depression and coma, tachycardia, cardiovascular collapse, seizures, and respiratory depression up to respiratory arrest. Fatal intoxications are rare and appear to be associated with large overdoses of tramadol.

According to the DEA (United States Drug Enforcement Administration), drugs, substances, and certain chemicals used to make drugs are classified into five (5) distinct categories or schedules depending upon the drug's acceptable medical use and the drug's abuse or dependency potential. The abuse rate is a determinate factor in the scheduling of the drug; for example, Schedule I drugs have a high potential for abuse and the potential to create severe psychological and/or physical dependence. As the drug schedule changes-- Schedule II, Schedule III, etc., so does the abuse potential-- Schedule V drugs represent the least potential for abuse. A Listing of drugs and their schedule are located at Controlled Substance Act (CSA) Scheduling or CSA Scheduling by Alphabetical Order. These lists describe the basic or parent chemical and do not necessarily describe the salts, isomers and salts of isomers, esters, ethers and derivatives which may also be classified as controlled substances. These lists are intended as general references and are not comprehensive listings of all controlled substances.

Tramadol is listed in Schedule IV listing drugs with a low potential for abuse and low risk of dependence.

In an official definition by the FDA, Drug products with abuse potential generally contain drug substances that have central nervous system (CNS) activity and produce euphoria (or other changes in mood), hallucinations, and effects consistent with CNS depressants or stimulants. Thus, if a drug substance is CNS-active, the new drug product containing that drug substance may need to undergo a thorough assessment of its abuse potential and may be subject to control, e.g. in the US under the Controlled Substances Act (CSA) (see generally 21 U.S.C. 811). Drug abuse is defined as the intentional, non-therapeutic use of a drug product or substance, even once, to achieve a desired psychological or physiological effect. Therefore, abuse potential refers to the likelihood that abuse will occur with a particular drug product or substance with CNS activity. Drug abuse is a serious public health problem that affects almost every community and family in some way. Each year drug abuse causes millions of serious illnesses or injuries among Americans. Abused drugs include metamphetamine, anabolic steroids, club drugs, cocaine, heroin, inhalants, marijuana, prescription drugs, including opioids. Drug abuse also plays a role in many major social problems, such as drugged driving, violence and stress. There are different types of treatments for drug abuse, one of them being to reduce the abuse potential of a drug by modifying its pharmacokinetics properties.

The World Health Organization (WHO) defines substance addiction as using a substance repeatedly, despite knowing and experiencing harmful effects. Substance addiction is a chronic, relapsing disease characterized by a loss of control over drug use, compulsive drug seeking and craving for a substance, use that persists despite negative consequences, and physical and/or psychological dependence on the substance. Substance abuse and addiction are public health issues.

On the other hand, opioid drugs such as tramadol are indispendsable in the clinical management of pain syndromes.

Thus, there is a constant and urgent need to find alternative or improved pharmacological activities in the treatment of pain, being effective while exhibiting a reduced abuse potential of the drug. This naturally also applies to tramadol, which is widely used as a relatively safe analgesic.

The following documents are to be cited:
- WO 2011/151080 A1 (ESTEVE LABOR DR.) (2011) (see below). It discloses further details of the formulations/pharmaceutical compositions disclosed below - also referring to E-58425.
- EP 2 586 432 A2 (ESTEVE LABOR DR.) (2013). It discloses further details of the formulations/pharmaceutical compositions disclosed below - also referring to E-58425.
- SEBASTIAN VIDELA et al., BRITISH JOURNAL OF CLINICAL PHARMACOLOGY., vol. 83, no. 12, (2017), pages 2718-2728. This article discloses the pharmacokinetics of the Co-Crystal of (rac)-tramadol HCl and Celecoxib (CTC) in a randomized open-label phase I clinical trial in healthy subjects.
- Helendooner et al., European Journal of Drug Metabolism and Pharmacokinetics, (2018), pages 63-75. This article discloses the pharmacokinetics of the Co-Crystalof (rac)-tramadol HCl and Celecoxib (CTC) in Japanese and Caucasian Subjects.
- EP 2 349 238 A1 (ESTEVE LABOR DR [ES]) (2011). It discloses further details of the formulations/pharmaceutical compositions disclosed below - also referring to E-58425.
- CARMEN ALMANSA et al.: CRYSTAL GROWTH & DESIGN., vol. 17, no. 4, 20 March (2017). This article discloses the Co-Crystalof (rac)-tramadol HCl and Celecoxib (CTC).
- JP 2017 190328 A (SHIONOGI & CO); (2017). It discloses a solid composition useful for preventing abuse of opioids.
- JOSE LOPEZ-CEDRUN et al., DRUGS IN RAND D, vol. 18, no. 2, (2018). This article discloses the Co-Crystal of (rac)-tramadol HCl and Celecoxib (CTC) in a randomized Phase II clinical trial.

### SUMMARY OF THE INVENTION:

The present invention is defined by the independent claims. The dependent claims depict other embodiments of the invention. Any embodiment not falling under the scope of the appended claims does not form part of the invention.

While investigating the potential of E-58425 in the treatment of pain, the applicant has highlighted that it lends itself to decrease the abuse liability of tramadol by modification of its pharmacokinetics properties.

The present invention is therefore related to the decrease of abuse liability of tramadol through the administration of a co-crystal of tramadol HCl and celecoxib. Thus, the application relates to a co-crystal of (rac)-tramadol·HCl and celecoxib for treating pain in a patient with an addiction to tramadol while reducing the abuse liability of tramadol.

It has surprisingly been found that co-crystals of tramadol and celecoxib like the co-crystal E-58425 treats pain while reducing the abuse liability compared to tramadol alone or to the combination of tramadol and celecoxib based on changes in its pharmacokinetic profile.

### DETAILED DESCRIPTION:

The attractiveness of opioids for abuse is usually evaluated using validated measures of subjective pharmacodynamic (PD) effects (e.g. drug liking, drug high), as well as selected pharmacokinetic characteristics of opioids, such as peak plasma concentration (Cₘₐₓ) and time to achieve Cₘₐₓ, expressed as Tₘₐₓ (Harris et al., Pain Medicine; 18: 1278-1291 (2017) and Kopecky et al.; The Journal of Clinical Pharmacology, 57(4) 500-512 (2017)). In terms of PK, the literature states that for opioids there is high correlation of the Cₘₐₓ/Tₘₐₓ ratio called Abuse Quotient (AQ) and PD effects, with higher AQ denoting greater abuse potential.

Taken in that context, Harris et al. evaluated the oral abuse potential and pharmacokinetics (PK) of hydrocodone intact, chewed, or milled to fine particles in comparison with hydrocodone solution or placebo. The results of the PK analysis indicate that the rate of hydrocodone absorption, illustrated by the calculation Cₘₐₓ/Tₘₐₓ, decreased in parallel with the PD measures of abuse potential. These differences may be associated with lower abuse potential because the rate of increase in concentration of opioid in plasma (Cₘₐₓ/Tₘₐₓ) is suggested to be positively correlated with the likelihood of abuse.

In Kopecky et al., it is described that the abuse quotient is a measure of average rate of rise in plasma concentration between dosing and Tₘₐₓ; the score is thought to be related to a product's abuse potential.

Harriet de Wit et al. (Psychopharmacology; 107: 352-358 (1992)) mentions that certain pharmacokinetic properties of drugs are believed to explain differences among drugs in liability for abuse. One of these properties is the rapidity with which the drug is delivered to the central nervous system. Different drugs within the same class are thought to differ in abuse liability because of this characteristic, and different routes of administration are thought to be associated with differential likelihood of abuse for similar reasons.

"Abuse liability" is defined as the propensity of a drug to produce compulsive use, to cause addiction, or the potential that a drug has for addiction. The term is used interchangeably with "potential for addiction".

"Abuse potential" or "potential for abuse" is the likelihood that abuse will occur with a particular drug product or substance with central nervous system activity.

"Abuse" is defined as the intentional non-therapeutic use of a drug, even once, to achieve a desired psychological or physiological effect. That is, the use of a drug in a way that is not intended or recommended. Abuse can lead to addiction.

"(Drug) Addiction" is defined as a compulsive drug use despite harmful consequences characterized by an inability to stop using a drug.

It seems therefore that abuse liability is higher for drugs with a high Cₘₐₓ and short Tₘₐₓ. The quotient Cₘₐₓ/Tₘₐₓ is determinant in how a drug is a potential for addiction: the lower the AQ the lower the abuse liability.

It has surprisingly been found that co-crystal E-58425 shows a better (lower) AQ ratio compared to Tramadol alone or to the combination of tramadol and celecoxib (administered as the commercially available drug products), leading to the conclusion that the co-crystal E-58425 treats pain while reducing the abuse liability or potential for addiction compared to tramadol alone or to the combination of tramadol and celecoxib.

The application relates in a first **Aspect** to a co-crystal of (rac)-tramadol·HCl and celecoxib for treating pain in a patient with an addiction to tramadol while reducing the abuse liability of tramadol.This treatment is done in a patient with an addiction to tramadol.

The application thus also relates in this major **Aspect A)** to a method for treating pain while reducing the abuse liability of tramadol, the method comprising administering to the subject a therapeutically effective dose of a co-crystal of (rac)-tramadol·HCl and celecoxib, or a pharmaceutically acceptable derivative thereof.

"Treating pain while reducing the abuse liability of tramadol" in the sense of this application is defined as treating a patient suffering from pain to ameliorate the syndrome by administering a therapeutically effective dose of the co-crystal while at the same time the potential for addiction of tramadol is reduced. In this sense it is understood, that preventing means that the risk of developing addiction when using the co-crystal is lowered compared to the risk if tramadol is used alone or in combination with celecoxib while having at least the same effect in treating pain

In a further **Aspect** the application also relates to a co-crystal of (rac)-tramadol·HCl and celecoxib, or a pharmaceutically acceptable derivative thereof, for treating pain in a patient with an addiction to tramadol or the risk of it.

The application thus also relates in this **Aspect** to a method for treating pain in a patient with an addiction to tramadol or the risk of it, the method comprising administering to the subject a therapeutically effective dose of a co-crystal of (rac)-tramadol·HCl and celecoxib, or a pharmaceutically acceptable derivative thereof. The application thus also relates in this **Aspect** to a method for treating pain in a patient with an addiction to tramadol, the method comprising administering to the subject a therapeutically effective dose of a co-crystal of (rac)-tramadol·HCl and celecoxib, or a pharmaceutically acceptable derivative thereof or to a method for treating pain in a patient at risk of an addiction to tramadol, the method comprising administering to the subject a therapeutically effective dose of a co-crystal of (rac)-tramadol·HCl and celecoxib, or a pharmaceutically acceptable derivative thereof.

"Treating pain in a patient with an addiction to tramadol" in the sense of this application is defined as treating a patient suffering from pain that is at the same time addicted to tramadol to ameliorate the syndrome by administering a therapeutically effective dose of the co-crystal. According to the WHO substance addiction is defined as using a substance repeatedly, despite knowing and experiencing harmful effects. Thus, this effect and indication can also be understood as meaning that the risk of developing (or aggravating/worsening) an addiction when using the co-crystal is lowered compared to the risk if tramadol is used alone or in combination with celecoxib while having at least the same effect in treating pain.

"Co-Crystal" as used herein is defined as a crystalline material comprising two or more compounds at ambient temperature (20 to 25°C, preferably 20°C), of which at least two are held together by weak interaction, wherein at least one of the compounds is a co-crystal former. Weak interaction is being defined as an interaction which is neither ionic nor covalent and includes for example: hydrogen bonds, van der Waals forces, and π-π interactions. Solvates of tramadol that do not further comprise a co-crystal former are not co-crystals according to the present invention. The co-crystals may however, include one or more solvate molecules in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself.

In scientific literature there currently is some discussion on the proper use of the word co-crystal (see for example Desiraju, Cryst. Eng. Comm., 2003, 5(82), 466-467 and Dunitz, Cryst. Eng. Comm., 2003, 5(91), 506). A recent article by Zawarotko (Zwarotko, Crystal Growth & Design, Vol. 7, No. 1, 2007, 4-9) gives a definition of co-crystal which is in line with the definition given above and thus also is a definition of "co-crystal" according to this invention. According to this article *"a co-crystal is a multiple component crystal in which all components are solid under ambient conditions when in their pure form. These components consist of a target molecule or ion and a molecular co-crystal former(s); when in a co-crystal, they coexist at a molecular level within a single crystal".*

In a preferred embodiment the application also relates to a co-crystal according to the invention wherein the molecular ratio between the (rac)-tramadol·HCl and celecoxib is 1:1.

E-58425 is a new co-crystal of tramadol HCl and celecoxib in a 1:1 molecular ratio. It appears as an example in WO2011/044962 A1. The overall profile of E-58425, with two relevant active principles for the treatment of pain for oral administration, suggests a unique and useful role in the management of moderate to severe pain.

The co-crystal E-58425 is formulated for oral administration containing as drug substance a co-crystal of racemic tramadol hydrochloride and celecoxib.

In a preferred embodiment the application also relates to the use of (or a method of treatments using) the co-crystal according to the invention comprising (rac)-tramadol·HCl and celecoxib in a molecular ratio of 1:1, wherein that the co-crystal shows a Powder X-Ray Diffraction pattern with at least one of the peaks [2θ] selected from the peaks [2θ] at 7.1, 9.3, 10.2, 10.7, 13.6, 13.9, 14.1, 15.5, 16.1, 16.2, 16.8, 17.5, 18.0, 19.0, 19.5, 19.9, 20.5, 21.2, 21.3, 21.4, 21.8, 22.1, 22.6, 22.7, 23.6, 24.1, 24.4, 25.2, 26.1, 26.6, 26.8, 27.4, 27.9, 28.1, 29.1, 29.9, 30.1, 31.1, 31.3, 31.7, 32.5, 32.8, 34.4, 35.0, 35.8, 36.2 and at 37.2[°].

The 2θ values are obtained using copper radiation (CuKα1 1.54060Å). The exact methods of determining this Powder X-Ray Diffraction pattern - which corresponds to the Powder X-Ray Diffraction pattern of E-58425 - can be found in the Example part and the figures of WO2011/044962 A1.

In another preferred embodiment the application also relates to the use of (or a method of treatments using) the co-crystal according to the invention comprising (rac)-tramadol·HCl and celecoxib in a molecular ratio of 1:1, wherein the co-crystal shows a Powder X-Ray Diffraction pattern with peaks [2θ] selected from:
- 14.1 and 22.7[°],
- 14.1 and 19.0[°],
- 14.1 and 16.8[°],
- 16.8 and 22.7[°],
- 16.8 and 19.0[°], and
- 19.0 and 22.7[°].

The 2θ values are obtained using copper radiation (CuKα1 1.54060Å). The exact methods of determining this Powder X-Ray Diffraction pattern - which corresponds to the Powder X-Ray Diffraction pattern of E-58425 - can be found in the Example part and the figures of WO2011/044962 A1.

In another preferred embodiment the application also relates to the use of (or a method of treatments using) the co-crystal according to the invention comprising (rac)-tramadol·HCl and celecoxib in a molecular ratio of 1:1, wherein the co-crystal shows a Powder X-Ray Diffraction pattern with peaks [2θ] selected from:
- 14.1, 16.8 and 22.7[°],
- 14.1, 19.0 and 22.7[°],
- 14.1, 16.8 and 19.0[°], and
- 16.8, 19.0 and 22.7[°].
The 2θ values are obtained using copper radiation (CuKα1 1.54060Å). The exact methods of determining this Powder X-Ray Diffraction pattern - which corresponds to the Powder X-Ray Diffraction pattern of E-58425 - can be found in the Example part and the figures of WO2011/044962 A1.

In another preferred embodiment the application also relates to the use of (or a method of treatments using) the co-crystal according to the invention comprising (rac)-tramadol·HCl and celecoxib in a molecular ratio of 1:1, wherein the co-crystal shows a Powder X-Ray Diffraction pattern with peaks [2θ] at 14.1, 16.8, 19.0 and 22.7[°]. In a further preferred embodiment of this embodiment the co-crystal shows additional peaks [2θ] at 7.1, 19.9 and 20.5[°] and in a further even more preferred embodiment of this preferred embodiment the co-crystal shows additional peaks [2θ] at 13.6, 13.9, 17.5, 18.0, 19.5, 21.2, 21.3, 21.8, 22.6, 23.6, 24.1, 24.4 and 26.1 [°].

The 2θ values are obtained using copper radiation (CuKα1 1.54060Å).. The exact methods of determining this Powder X-Ray Diffraction pattern - which corresponds to the Powder X-Ray Diffraction pattern of E-58425 - can be found in the Example part and the figures of WO2011/044962 A1.

In another preferred embodiment the application also relates to the use of (or a method of treatments using) the co-crystal according to the invention comprising (rac)-tramadol·HCl and celecoxib in a molecular ratio of 1:1, wherein the co-crystal shows a Powder X-Ray Diffraction pattern with peaks [2θ] at 7.1, 9.3, 10.2, 10.7, 13.6, 13.9, 14.1, 15.5, 16.1, 16.2, 16.8, 17.5, 18.0, 19.0, 19.5, 19.9, 20.5, 21.2, 21.3, 21.4, 21.8, 22.1, 22.6, 22.7, 23.6, 24.1, 24.4, 25.2, 26.1, 26.6, 26.8, 27.4, 27.9, 28.1, 29.1, 29.9, 30.1, 31.1, 31.3, 31.7, 32.5, 32.8, 34.4, 35.0, 35.8, 36.2 and 37.2[°].

The 2θ values are obtained using copper radiation (CuKα1 1.54060Å). The exact methods of determining this Powder X-Ray Diffraction pattern - which corresponds to the Powder X-Ray Diffraction pattern of E-58425 - can be found in the Example part and the figures of WO2011/044962 A1.

In another preferred embodiment the application also relates to the use of (or a method of treatments using) the co-crystal according to the invention comprising (rac)-tramadol·HCl and celecoxib in a molecular ratio of 1:1, wherein the co-crystal shows a Fourier Transform Infra Red pattern with absorption bands at 3481.6 (m), 3133.5 (m), 2923.0 (m), 2667.7 (m), 1596.0 (m), 1472.4 (m), 1458.0 (m), 1335.1 (m), 1288.7 (m), 1271.8 (m), 1168.7 (s), 1237.3 (m), 1168.7 (s), 1122.6 (s), 1100.9 (m), 1042.2 (m), 976.8 (m), 844.6 (m), 820.1 (m), 786.5 (m) 625.9 (m) cm-1.

The exact methods of determining this Fourier Transform Infra Red pattern - which corresponds to the Fourier Transform Infra Red pattern of E-58425 - can be found in the Example part and the figures of WO2011/044962 A1.

In another preferred embodiment the application also relates to the use of (or a method of treatments using) the co-crystal according to the invention comprising (rac)-tramadol·HCl and celecoxib in a molecular ratio of 1:1, wherein the co-crystal has an orthorhombic unit cell with the following dimensions:
a = 11.0323(7) Å
b = 18.1095(12) Å
c = 17.3206(12) Å.
The exact methods of determining the nature and dimensions of the co-crystal - which corresponds to the nature and dimensions of E-58425 - can be found in the Example part and the figures of WO2011/044962 A1.

In another preferred embodiment the application also relates to the use of (or a method of treatments using) the co-crystal according to the invention comprising (rac)-tramadol·HCl and celecoxib in a molecular ratio of 1:1, wherein for the co-crystal the endothermic sharp peak corresponding to the melting point has an onset at 164°C.

The exact methods of determining the onset of the endothermic sharp peak corresponding to the melting point of the co-crystal - which corresponds to the onset of the endothermic sharp peak corresponding to the melting point of E-58425 - can be found in the Example part and the figures of WO2011/044962 A1.

In another preferred embodiment the application also relates to the use of (or a method of treatments using) the co-crystal according to the invention for the treatment of acute pain, chronic pain, neuropathic pain, nociceptive pain, mild and severe to moderate pain, hyperalgesia, pain related to central sensitization, allodynia or cancer pain, including diabetic neuropathy or diabetic peripheral neuropathy and osteoarthritis, fibromyalgia; rheumatoid arthritis, ankylosing spondylitis, frozen shoulder or sciatica.

In another preferred embodiment the application also relates to the use of (or a method of treatments using) the co-crystal according to the invention for the treatment of acute and chronic moderate to severe pain, acute moderate to severe pain, acute moderate pain, acute severe pain, chronic moderate to severe pain, chronic moderate pain, or chronic severe pain.

"Pain" is defined by the International Association for the Study of Pain (IASP) as "an unpleasant sensory and emotional experience associated with actual or potential tissue damage, or described in terms of such damage (IASP, Classification of chronic pain, 2^{nd} Edition, IASP Press (2002), 210). Even though pain is always subjective its causes or syndromes can be classified. One classification to denominate subtypes of pain would be to divide the general pain syndrome into the subtypes of acute and chronic pain or - according to the pain intensity - into mild, moderate and severe pain. In other definitions the general pain syndrome is also divided into "nociceptive" (caused by activation of nociceptors), "neuropathic" (caused by damage to or malfunction of the nervous system) and pain related to central sensitization (central pain syndrome).

According to the IASP "allodynia" is defined as "a pain due to a stimulus which does not normally provoke pain" (IASP, Classification of chronic pain, 2nd Edition, IASP Press (2002), 210). Even though the symptoms of allodynia are most likely associated as symptoms of neuropathic pain this is not necessarily the case so that there are symptoms of allodynia not connected to neuropathic pain though rendering allodynia in some areas broader than neuropathic pain.

The IASP further draws the following difference between "allodynia", "hyperalgesia" and "hyperpathia" (IASP, Classification of chronic pain, 2nd Edition, IASP Press (2002), 212):

| Allodynia | Lowered threshold | Stimulus and response mode differ |
|---|---|---|
| Hyperalgesia | Increased response | Stimulus and response rate are the same |
| Hyperpathia | Raised threshold; Increased response | Stimulus and response rate may be the same or different |

According to the IASP "neuropathy" is defined as "a primary lesion or dysfunction in the nervous system" (IASP, Classification of chronic pain, 2nd Edition, IASP Press (2002), 211). Neuropathic pain may have central or peripheral origin.

"Sciatica" or "sciatic neuritis is defined herein as a set of symptoms including pain that derive from irritation of the sciatic nerve or its roots,
"Frozen shoulder" or "adhesive capsulitis" is defined herein as a symptom wherein the connective tissue surrounding the shoulder joint or the shoulder capsule itself, is causing chronic pain, becoming inflamed and stiff.

"Ankylosing spondylitis" or "Morbus Bechterew" is a chronic, inflammatory arthritis and autoimmune disease. It mainly affects joints in the spine and the sacroilium in the pelvis, causing eventual fusion of the spine.

"Pain related to central sensitization" / "central pain syndrome" is defined within this application as a neurological condition caused by damage to or dysfunction of the central nervous system (CNS), which includes the brain, brainstem, and spinal cord. This syndrome can *inter alia* be caused by stroke, multiple sclerosis, tumors, epilepsy, brain or spinal cord trauma, or Parkinson's disease.

"Nociceptive pain " is defined as a type of pain caused by activation of nociceptors. It can be divided into somatic and visceral pain. "Visceral pain" is pain generally originating from the organs, whereas "(deep) somatic pain" originates from ligaments, tendons, bones, blood vessels, fasciae and muscles.

In another preferred embodiment the application also relates to the use of (or a method of treatments using) the co-crystal according to the invention, wherein the co-crystal is comprised in a pharmaceutical composition also comprising at least a solubility enhancer polymer; wherein the solubility enhancer polymer is selected from polyvinyl caprolactam-polyvinyl acetate-polyethylene glycol graft copolymer or from copovidone, povidone, cyclodextrin, polyethylene glycol and lauroyl macrogol-32 glycerides EP, preferably wherein the solubility enhancer polymer is selected from polyvinyl caprolactam-polyvinyl acetate-polyethylene glycol graft copolymer or other hydrophilic polymers selected from copovidone, or povidone, most preferably wherein the solubility enhancer polymer is copovidone.

Further details of the formulations/pharmaceutical compositions can be found - also referring to E-58425 - in WO2011/151080 A1 (see above).

The present invention is illustrated below with the help of the following figures and examples. These illustrations are given solely by way of example and do not limit the invention.

### Brief description of the figures:

**Figure 1****:**
   Linear profile of the mean plasma concentrations for tramadol and O-desmethyltramadol after oral administration to healthy male and female subjects of Co-crystal E-58425 (Treatment-1), Ultram^{®} (Treatment-2) and Ultram ^{®} + Celebrex^{®} (Treatment-4).
**Figure 2****:**
   Abuse Quotient (AQ) for tramadol and its metabolite M1, obtained from Co-crystal E-58425 (Treatment-1) Ultram^{®} (Treatment-2) and Ultram ^{®} + Celebrex^{®} (Treatment-4) calculated from the data obtained in a 4-way cross-over study. For opioids, there is high correlation of the Cₘₐₓ/Tₘₐₓ ratio with pharmacodynamic effects, with higher Cₘₐₓ/Tₘₐₓ (faster and/or higher absorption) denoting greater abuse potential.

For Co-crystal E-58425, the AQs obtained for tramadol in the 4-way cross-over study were lower than those obtained for Ultram^{®} and for Ultram^{®} and Celebrex^{®} taken concomitantly and these differences were statistically significant, suggesting a lower abuse potential for Co-crystal. No statistically significant differences were observed for Ultram^{®} taken alone or in combination with Celebrex^{®}.

### Pharmacokinetic Parameter Definitions

Cₘₐₓ Maximum observed plasma concentration
Tₘₐₓ Time of maximum observed plasma concentration
AQ Abuse quotient
HPLC High performance liquid chromatography
MS/MS Triple quadrupole mass spectrometry
IS1/ISTD1 Internal standard (tramadol-D6)
IS2/ISTD2 Internal standard (O-desmethyltramadol-D6)
K₂-EDTA Di-potassium ethylene diamine tetraacetic acid
LOQ Lower Limit of Quantitation
M1: O-desmethyltramadol
S.D. Standard Deviation
SE Standarf Error
CV Coefficient variation
mg Milligrams
mL Milliliters
ng Nanograms

### EXAMPLES

The study was a single center, randomized, single dose, open-label, 4-period, 4-sequence, crossover design in healthy male and female subjects. The following treatments were administered under fasting conditions:
**Treatment-1:** 2 x 100 mg Co-crystal E-58425 tablets, administered alone
**Treatment-2:** 2 x 50 mg Tramadol HCl tablets (Ultram^{®}), administered alone
**Treatment-3:** 1 x 100 mg Celecoxib capsule (Celebrex^{®}), administered alone (data not shown)
**Treatment-4:** 2 x 50 mg Tramadol tablets (Ultram^{®}) and 1 x 100 mg Celecoxib capsule (Celebrex^{®}), taken concomitantly.

The products were administered to 36 subjects. The wash-out between the periods for each subject was of 7 calendar days.

**Table 1. Study Sequences**

| | **Period 1** | **Period 2** | **Period 3** | **Period 4** |
|---|---|---|---|---|
| Sequence 1 (n= 9) | Treatment-1 | Treatment-2 | Treatment-4 | Treatment-3 |
| Sequence 2 (n= 9) | Treatment-2 | Treatment-3 | Treatment-1 | Treatment-4 |
| Sequence 3 (n= 9) | Treatment-3 | Treatment-4 | Treatment-2 | Treatment-1 |
| Sequence 4 (n= 9) | Treatment-4 | Treatment-1 | Treatment-3 | Treatment-2 |

### Handling of Samples

Separate blood samples were obtained for analysis of tramadol / M1 metabolite and for analysis of celecoxib (data not shown). Blood samples for quantification of tramadol and M1 metabolite (Treatment-1, Treatment-2 and Treatment-4) were collected prior to drug administration and at 0.5, 0.75, 1, 1.25, 1.5, 1.75, 2, 2.5, 3, 3.5, 4, 6, 8, 12, 24, 36 and 48 hours after drug administration in K₂-EDTA Vacutainers.

As soon as possible following blood collection, samples were centrifuged at a temperature of 4°C nominal and at approximately 1500g for 10 minutes. The plasma obtained was separated into duplicate polypropylene culture tubes. Each tube was labeled in order to identify the drug to be assayed and with a code number that did not reveal formulation identity. The samples were frozen in an upright position and retained in the clinic's freezers at a temperature of -20°C nominal until sent on dry ice to the bioanalytical facility for assay.

### Method of Measurement

The experimental human plasma samples were assayed for tramadol and M1 metabolite (O-desmethyltramadol) and also for celecoxib (data not shown) using two different validated bioanalytical methods by HPLC with MS/MS detection.

Sample pre-treatment involved the liquid-liquid extraction of tramadol and O-desmethyltramadol from 0.100 mL of human plasma; tramadol-D6 and O-desmethyltramadol-D6 were used as the internal standards (IS1 and IS2 respectively). The compounds were identified and quantified using Hilic HPLC with MS/MS detection over a theoretical concentration range of 2.00 ng/mL to 800.00 ng/mL for tramadol and 0.500 ng/mL to 200.00 ng/mL for O-desmethyltramadol. The concentrations were calculated using peak area ratios and the linearity of the calibration curve was determined using a weighted (1/x²) linear (y=mx+b) least squares regression analysis for tramadol and for O-desmethyltramadol.

### Pharmacokinetic analysis

Pharmacokinetic parameters were estimated from the individual plasma concentration - time profile for each subject. The main absorption and disposition parameters were obtained using a non-compartmental approach with a log-linear terminal phase assumption. Maximum observed plasma concentration (Cₘₐₓ) and the time of maximum observed plasma concentration (Tₘₐₓ) were obtained directly from the experimental data. The trapezoidal rule was used to estimate the area under the curve (AUC; data not shown) and the terminal phase was estimated by maximizing the coefficient of determination estimated from the log-linear regression model (T_{1/2}; data not shown). The abuse quotient was obtained using the following expression AQ= Cₘₐₓ/Tₘₐₓ.

### RESULTS

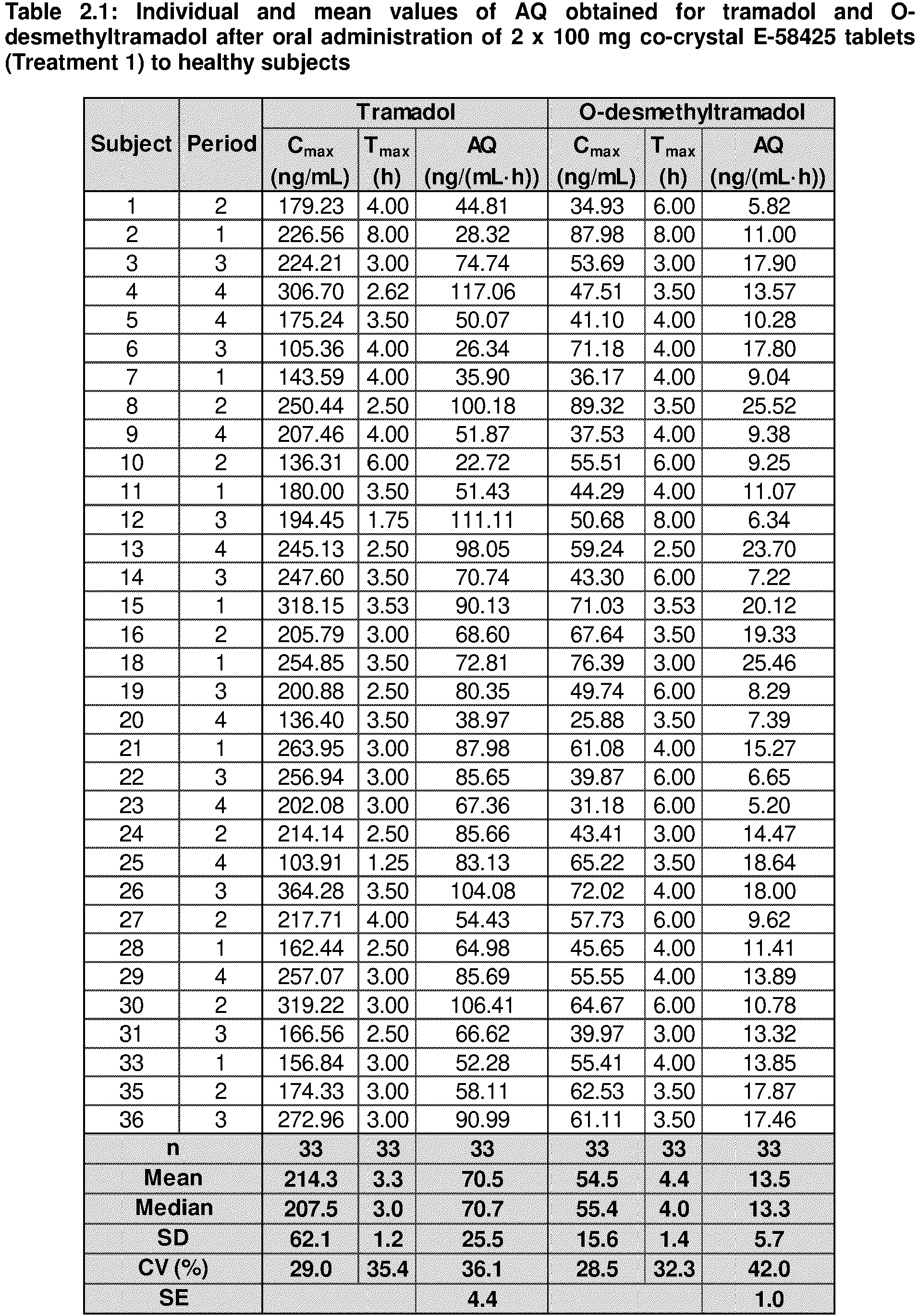

Subjects 17, 32 and 34 withdrew their consent or were withdrawn from the study

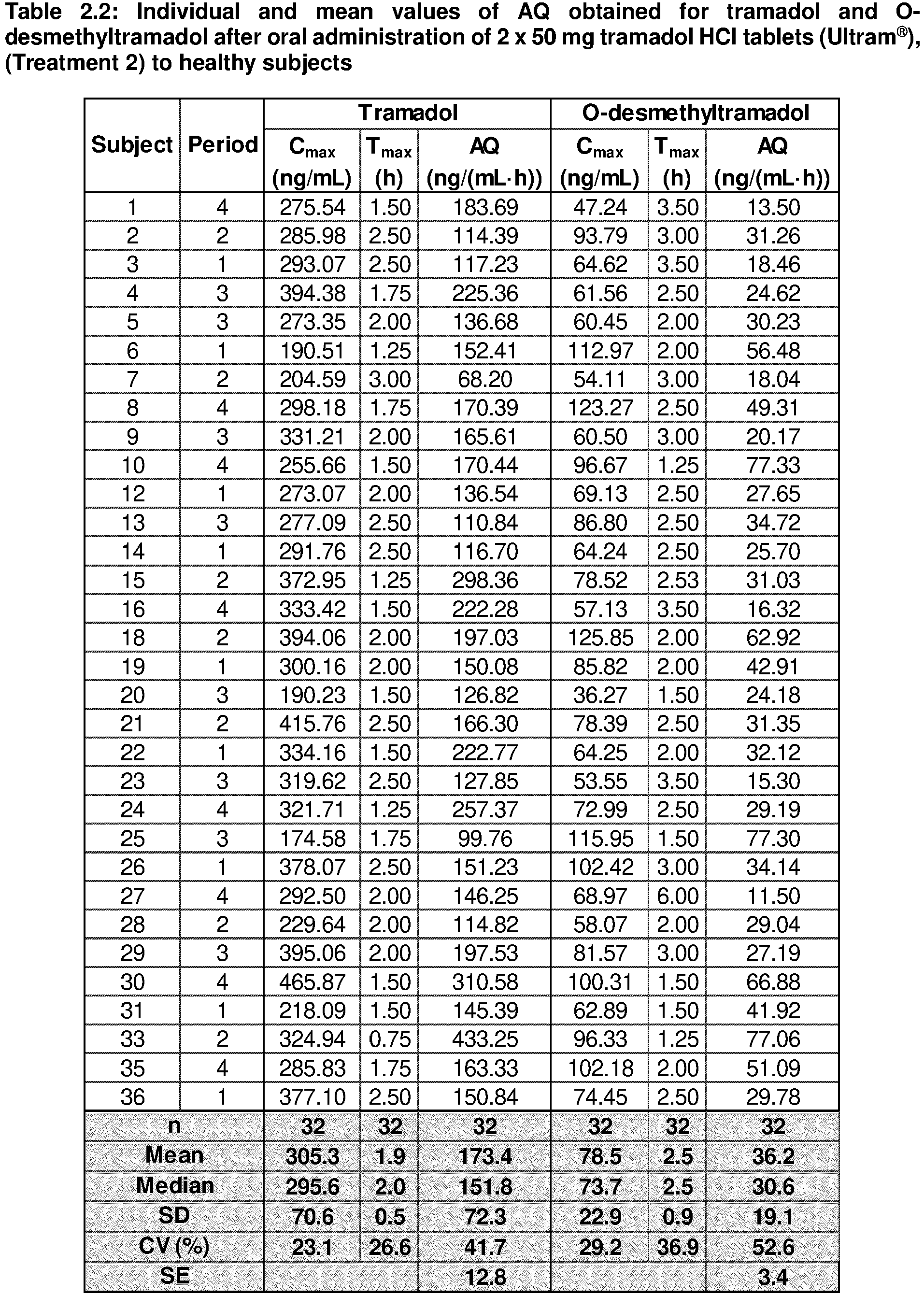

Subjects 11, 17, 32 and 34 withdrew their consent or were withdrawn from the study

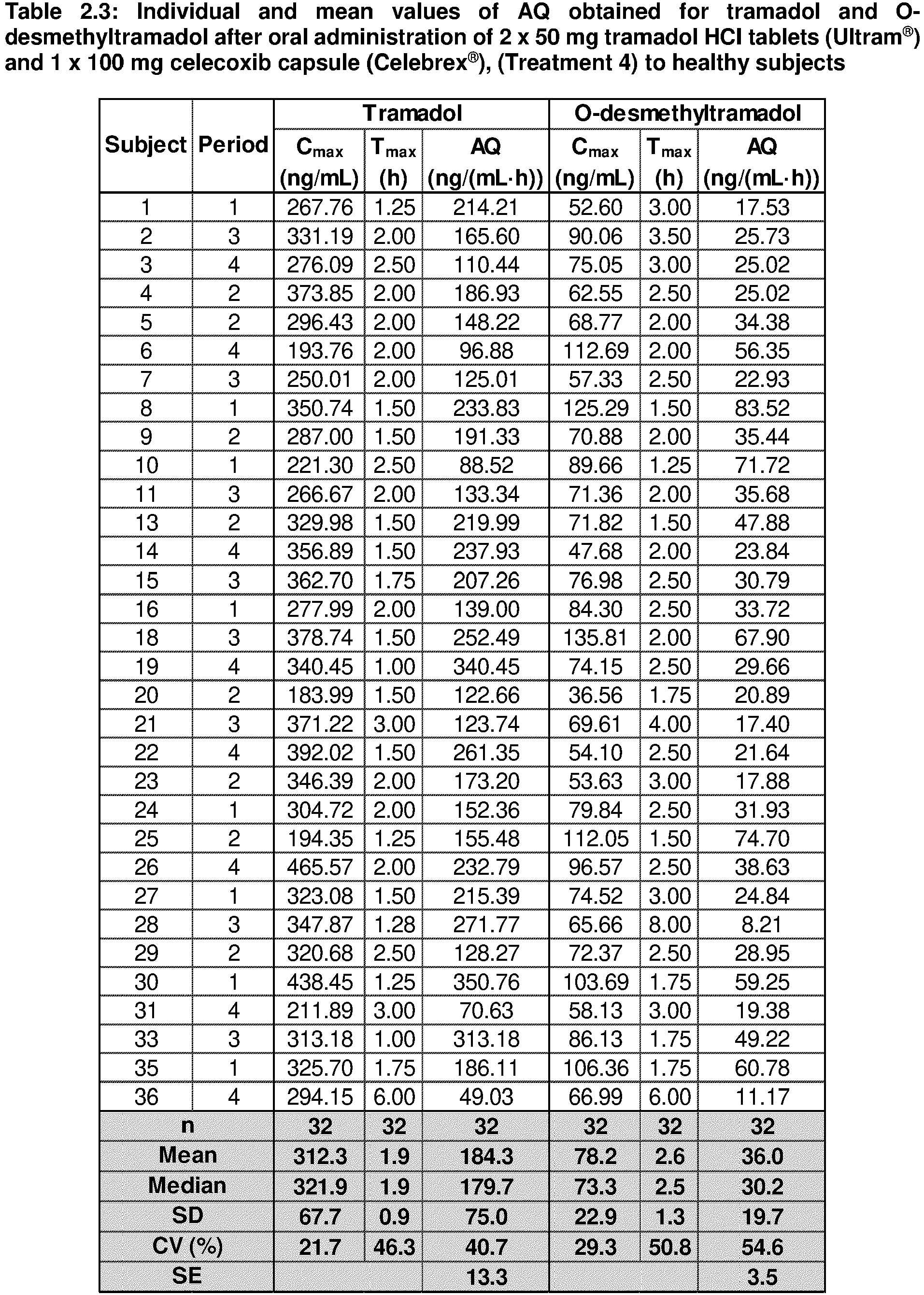

Subjects 12, 17, 32 and 34 withdrew their consent or were withdrawn from the study

**Table 3: Summary of AQ obtained for tramadol and O-desmethyltramadol (M1) after oral administration of co-crystal E-58425 tablets, 2 x 50 mg tramadol HCl tablets (Ultram^{®}) and 2 x 50 mg tramadol HCl tablets (Ultram^{®}), and 1 x 100 mg celecoxib capsule (Celebrex^{®}) to healthy subjects**

| **AQ** | **Co-crystal E-58425** | | **Ultram^{R}** | | **Ultram^{R}+ Celebrex^{R}** | |
|---|---|---|---|---|---|---|
| | **Mean** | **SE** | **Mean** | **SE** | **Mean** | **SE** |
| Tramadol | 70.5 | 4.4 | 173.4 | 12.8 | 184.3 | 13.3 |
| M1 | 13.5 | 1.0 | 36.2 | 3.4 | 36.0 | 3.5 |

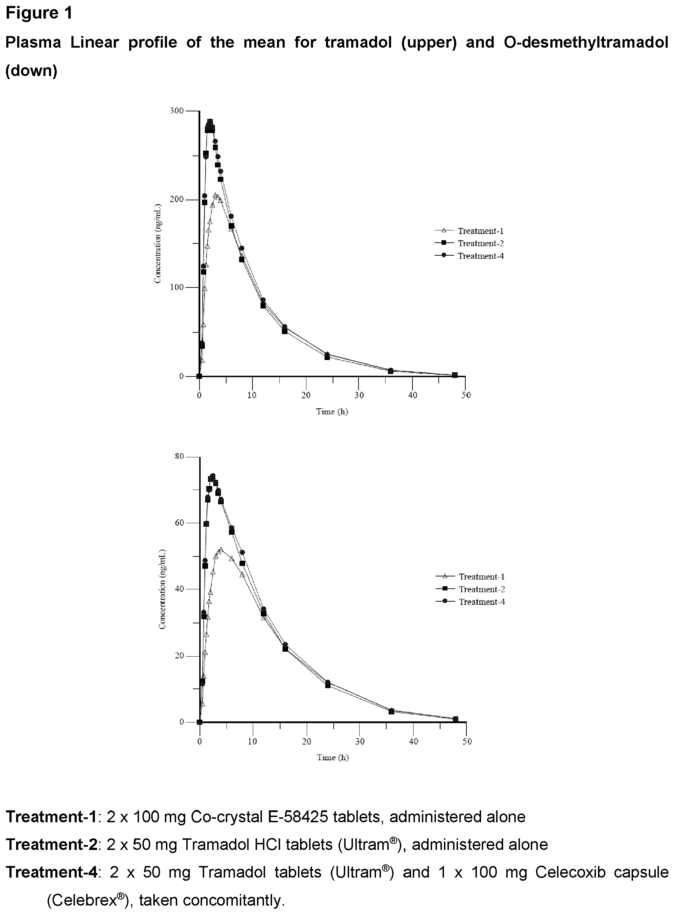

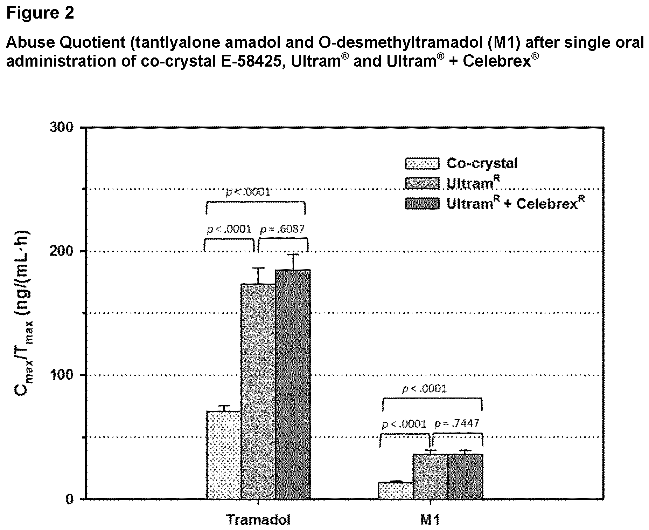

## Claims

1. A co-crystal of (rac)-tramadol·HCl and celecoxib, for use in the treatment of pain in a patient with an addiction to tramadol while reducing the abuse liability of tramadol.

2. The co-crystal for use according to claim 1, wherein the molecular ratio between the (rac)-tramadol·HCl and celecoxib is 1:1.

3. The co-crystal for use according to any one of claims 1 or 2 comprising (rac)-tramadol·HCl and celecoxib in a molecular ratio of 1:1, **characterized in that** the co-crystal shows a Powder X-Ray Diffraction pattern with at least one of the peaks [2θ] selected from 7.1, 9.3, 10.2, 10.7, 13.6, 13.9, 14.1, 15.5, 16.1, 16.2, 16.8, 17.5, 18.0, 19.0, 19.5, 19.9, 20.5, 21.2, 21.3, 21.4, 21.8, 22.1, 22.6, 22.7, 23.6, 24.1, 24.4, 25.2, 26.1, 26.6, 26.8, 27.4, 27.9, 28.1, 29.1, 29.9, 30.1, 31.1, 31.3, 31.7, 32.5, 32.8, 34.4, 35.0, 35.8, 36.2 and 37.2[°], with the 2θ values being obtained using copper radiation (Cu_{Kα1} 1.54060Å).

4. The co-crystal for use according to any one of claims 2 or 3, **characterized in that** the co-crystal shows a Powder X-Ray Diffraction pattern with peaks [2θ] at 14.1, 16.8, 19.0 and 22.7[°], with the 2θ values being obtained using copper radiation (CuKα1 1.54060Å).

5. The co-crystal for use according to claim 4, **characterized in that** the co-crystal shows additional peaks [2θ] at 7.1, 19.9 and 20.5[°].

6. The co-crystal for use according to claim 5, **characterized in that** the co-crystal shows additional peaks [2θ] at 13.6, 13.9, 17.5, 18.0, 19.5, 21.2, 21.3, 21.8, 22.6, 23.6, 24.1, 24.4 and 26.1 [°].

7. The co-crystal for use according to any one of claims 1 to 6 comprising (rac)-tramadol·HCl and celecoxib in a molecular ratio of 1:1, **characterized in that** the co-crystal shows a Powder X-Ray Diffraction pattern with peaks [2θ] selected from at 7.1, 9.3, 10.2, 10.7, 13.6, 13.9, 14.1, 15.5, 16.1, 16.2, 16.8, 17.5, 18.0, 19.0, 19.5, 19.9, 20.5, 21.2, 21.3, 21.4, 21.8, 22.1, 22.6, 22.7, 23.6, 24.1, 24.4, 25.2, 26.1, 26.6, 26.8, 27.4, 27.9, 28.1, 29.1, 29.9, 30.1, 31.1, 31.3, 31.7, 32.5, 32.8, 34.4, 35.0, 35.8, 36.2 and 37.2[°], with the 2θ values being obtained using copper radiation (Cu_{Kα1} 1.54060Å).

8. The co-crystal for use according to any one of claims 1 to 7 comprising (rac)-tramadol·HCl and celecoxib in a molecular ratio of 1:1, **characterized in that** the co-crystal shows a Fourier Transform Infra Red pattern with absorption bands at 3481.6 (m), 3133.5 (m), 2923.0 (m), 2667.7 (m), 1596.0 (m), 1472.4 (m), 1458.0 (m), 1335.1 (m), 1288.7 (m), 1271.8 (m), 1168.7 (s), 1237.3 (m), 1168.7 (s), 1122.6 (s), 1100.9 (m), 1042.2 (m), 976.8 (m), 844.6 (m), 820.1 (m), 786.5 (m) 625.9 (m) cm⁻¹.

9. The co-crystal for use according to any one of claims 1 to 8 comprising (rac)-tramadol·HCl and celecoxib in a molecular ratio of 1:1, **characterized in that** the co-crystal has an orthorhombic unit cell with the following dimensions:
a = 11.0323(7) Å
b = 18.1095(12) Å
c = 17.3206(12) Å.

10. The co-crystal for use according to any one of claims 1 to 9 comprising (rac)-tramadol·HCl and celecoxib in a molecular ratio of 1:1, **characterized in that** for the co-crystal the endothermic sharp peak corresponding to the melting point has an onset at 164°C.

11. The co-crystal for use according to any one of claims 1 to 10, in the treatment of acute pain, chronic pain, neuropathic pain, nociceptive pain, mild and severe to moderate pain, hyperalgesia, pain related to central sensitization, allodynia or cancer pain, including diabetic neuropathy or diabetic peripheral neuropathy and osteoarthritis, fibromyalgia; rheumatoid arthritis, ankylosing spondylitis, frozen shoulder or sciatica.

12. The co-crystal for use according to any one of claims 1 to 11 wherein the pain is acute and chronic moderate to severe pain, acute moderate to severe pain, acute moderate pain, acute severe pain, chronic moderate to severe pain, chronic moderate pain, or chronic severe pain.

13. The co-crystal for use according to any one of claims 1 to 12 wherein the co-crystal is comprised in a pharmaceutical composition also comprising at least a solubility enhancer polymer; wherein the solubility enhancer polymer is selected from polyvinyl caprolactam-polyvinyl acetate-polyethylene glycol graft copolymer or from copovidone, povidone, cyclodextrin, polyethylene glycol and lauroyl macrogol-32 glycerides EP, preferably wherein the solubility enhancer polymer is selected from polyvinyl caprolactam-polyvinyl acetate-polyethylene glycol graft copolymer or other hydrophilic polymers selected from copovidone, or povidone, most preferably wherein the solubility enhancer polymer is copovidone.

## Patentansprüche

1. Co-Kristall aus (rac)-Tramadol·HCl und Celecoxib zur Verwendung bei der Schmerzbehandlung bei Patienten mit einer Abhängigkeit von Tramadol und gleichzeitiger Verringerung der Missbrauchsanfälligkeit von Tramadol.

2. Co-Kristall zur Verwendung nach Anspruch 1, wobei das Molverhältnis zwischen dem (Rac)-Tramadol-HCl und Celecoxib 1:1 beträgt

3. Co-Kristall zur Verwendung nach einem der Ansprüche 1 oder 2, der (rac)-Tramadol-HCl und Celecoxib in einem Molverhältnis von 1:1 umfasst, **dadurch gekennzeichnet, dass** der Co-Kristall ein Pulverröntgendiffraktionsmuster mit wenigstens einem der Peaks [2θ] bei ausgewält aus 7,1, 9,3, 10,2, 10,7, 13,6, 13,9, 14,1, 15,5, 16,1, 16,2, 16,8, 17,5, 18,0, 19,0, 19,5, 19,9, 20,5, 21,2, 21,3, 21,4, 21,8, 22,1, 22,6, 22,7, 23,6, 24,1, 24,4, 25,2, 26,1, 26,6, 26,8, 27,4, 27,9, 28,1, 29,1, 29,9, 30,1, 31,1, 31,3, 31,7, 32.5, 32,8, 34,4, 35,0, 35,8, 36,2 und 37,2[°] aufweist, wobei die 2θ-Werte unter Verwendung von Kupferstrahlung (Cu_{Kα1} 1,54060Å) erhalten werden.

4. Co-Kristall zur Verwendung nach einem der Ansprüche 2 oder 3, **dadurch gekennzeichnet, dass** der Co-Kristall ein Pulverröntgendiffraktionsmuster mit Peaks bei 14,1, 16,8, 19,0 und 22,7[°] aufweist, wobei die 2θ-Werte unter Verwendung von Kupferstrahlung (Cu_{Kα1} 1,54060Å) erhalten werden.

5. Co-Kristall zur Verwendung nach Anspruch 4, **dadurch gekennzeichnet, dass** der Co-Kristall zusätzliche Peaks [2θ] bei 7,1, 19,9 und 20,5[°] aufweist.

6. Co-Kristall zur Verwendung nach Anspruch 5, **dadurch gekennzeichnet, dass** der Co-Kristall zusätzliche Peaks [2θ] bei 13,6, 13,9, 17,5, 18,0, 19,5, 21,2, 21,3, 21,8, 22,6, 23,6, 24,1, 24,4 und 26,1 [°] aufweist.

7. Co-Kristall zur Verwendung nach einem der Ansprüche 1 bis 6, der (rac)-Tramadol-HCl und Celecoxib in einem Molverhältnis von 1:1 umfasst, **dadurch gekennzeichnet, dass** der Co-Kristall ein Pulverröntgendiffraktionsmuster mit Peaks [2θ] bei ausgewält aus 7,1, 9,3, 10,2, 10,7, 13,6, 13,9, 14,1, 15,5, 16,1, 16,2, 16,8, 17,5, 18,0, 19,0, 19,5, 19,9, 20,5, 21,2, 21,3, 21,4, 21,8, 22,1, 22,6, 22,7, 23,6, 24,1, 24,4, 25,2, 26,1, 26,6, 26,8, 27,4, 27,9, 28,1, 29,1, 29,9, 30,1, 31,1, 31,3, 31,7, 32,5, 32,8, 34,4, 35,0, 35,8, 36,2 und 37,2[°] aufweist, wobei die 2θ-Werte unter Verwendung von Kupferstrahlung (Cu_{Kα1} 1,54060Å) erhalten werden.

8. Co-Kristall zur Verwendung nach einem der Ansprüche 1 bis 7, der (rac)-Tramadol-HCl und Celecoxib in einem Molverhältnis von 1:1 umfasst, **dadurch gekennzeichnet, dass** es ein Fourier-Transformations-Infrarotmuster mit Absorptionsbanden bei 3481,6 (m), 3133,5 (m), 2923,0 (m), 2667,7 (m), 1596,0 (m), 1472,4 (m), 1458,0 (m), 1335,1 (m), 1288,7 (m), 1271,8 (m), 1168,7 (s), 1237,3 (m), 1168,7 (s), 1122,6 (s), 1100,9 (m), 1042,2 (m), 976,8 (m), 844,6 (m), 820,1 (m), 786,5 (m) 625,9 (m) cm⁻¹.aufweist.

9. Co-Kristall zur Verwendung nach einem der Ansprüche 1 bis 8, der (rac)-Tramadol-HCl und Celecoxib in einem Molverhältnis von 1:1 umfasst, **dadurch gekennzeichnet, dass** der Co-Kristall eine orthorhombische Einheitszelle mit den folgenden Dimensionen aufweist:
a = 11,0323(7) Å
b = 18,1095(12) Å
c = 17,3206(12) Å.

10. Co-Kristall zur Verwendung nach einem der Ansprüche 1 bis 9, der (rac)-Tramadol-HCl und Celecoxib in einem Molverhältnis von 1:1 umfasst, **dadurch gekennzeichnet, dass** für den Co-Kristall der endotherme scharfe Peak, der dem Schmelzpunkt entspricht, bei 164 °C einsetzt.

11. Co-Kristall zur Verwendung nach einem der Ansprüche 1 bis 10, in der Behandlug von akuten Schmerzen, chronischen Schmerzen, neuropathischen Schmerzen, nozizeptiven Schmerzen, milden und starken bis mäßigen Schmerzen, Hyperalgesie, mit zentraler Sensibilisierung verbundenen Schmerzen, Allodynie oder Krebsschmerzen, einschließlich diabetischer Neuropathie oder diabetischer peripherer Neuropathie und Osteoarthritis, Fibromyalgie; rheumatoider Arthritis, Spondilitis ankylosans, Schultersteife oder Ischias.

12. Co-Kristall zur Verwendung nach einem der Ansprüche 1 bis 11, wobei der Schmerz akuter und chronischer mäßiger bis starker Schmerz, akuter mäßiger bis starker Schmerz, akuter mäßiger Schmerz, akuter starker Schmerz, chronischer mäßiger bis starker Schmerz, chronischer mäßiger Schmerz, oder chronischer starker Schmerz ist.

13. Co-Kristall zur Verwendung nach einem der Ansprüche 1 bis 12, wobei der Co-Kristall in einer pharmazeutischen Zusammensetzung enthalten ist, die auch wenigstens ein Löslichkeitsverstärkerpolymer umfasst; wobei das Löslichkeitsverstärkerpolymer ausgewählt ist aus Polyvinylcaprolactam-Polyvinylacetat-Polyethylenglykol Pfropfcopolymer oder aus Copovidon, Povidon, Cyclodextrin, Polyethylenglykol und Lauroyl-Macrogol-32-glyceriden EP, vorzugsweise wobei das Löslichkeitsverstärkerpolymer ausgewählt ist aus Polyvinylcaprolactam-Polyvinylacetat-Polyethylenglykol Pfropfcopolymer oder anderen hydrophilen Polymeren, ausgewählt aus Copovidon oder Povidon, besonders vorzugsweise wobei das Löslichkeitsverstärkrpolymer Copovidon ist.

## Revendications

1. Co-cristal de (rac)-tramadol•HCl et de célécoxib, pour une utilisation dans le traitement de la douleur chez un patient présentant une addiction au tramadol tout en réduisant le risque d'abus de tramadol.

2. Co-cristal pour une utilisation selon la revendication 1, dans lequel le rapport moléculaire entre le (rac)-tramadol•HCl et le célécoxib est de 1/1.

3. Co-cristal pour une utilisation selon l'une quelconque des revendications 1 ou 2, comprenant du (rac)-tramadol•HCl et du célécoxib en un rapport moléculaire de 1/1, **caractérisé en ce que** le co-cristal présente un motif de diffraction des rayons X sur poudre ayant au moins l'un des pics [2θ] choisis parmi 7,1, 9,3, 10,2, 10,7, 13,6, 13,9, 14,1, 15,5, 16,1, 16,2, 16,8, 17,5, 18,0, 19,0, 19,5, 19,9, 20,5, 21,2, 21,3, 21,4, 21,8, 22,1, 22,6, 22,7, 23,6, 24,1, 24,4, 25,2, 26,1, 26,6, 26,8, 27,4, 27,9, 28,1, 29,1, 29,9, 30,1, 31,1, 31,3, 31,7, 32,5, 32,8, 34,4, 35,0, 35,8, 36,2 et 37,2 [°], les valeurs de 2θ étant obtenues par utilisation du rayonnement du cuivre (Cu_{Kα1} 1,54060 Å).

4. Co-cristal pour une utilisation selon l'une quelconque des revendications 2 ou 3, **caractérisé en ce que** le co-cristal présente un motif de diffraction des rayons X sur poudre ayant des pics [2θ] à 14,1, 16,8, 19,0 et 22,7 [°], les valeurs de 2θ étant obtenues par utilisation du rayonnement du cuivre (CuKα1 1,54060 Å).

5. Co-cristal pour une utilisation selon la revendication 4, **caractérisé en ce que** le co-cristal présente des pics additionnels [2θ] à 7,1, 19,9 et 20,5 [°].

6. Co-cristal pour une utilisation selon la revendication 5, **caractérisé en ce que** le co-cristal présente des pics additionnels [2θ] à 13,6, 13,9, 17,5, 18,0, 19,5, 21,2, 21,3, 21,8, 22,6, 23,6, 24,1, 24,4 et 26,1 [°].

7. Co-cristal pour une utilisation selon l'une quelconque des revendications 1 à 6, comprenant du (rac)-tramadol•HCl et du célécoxib en un rapport moléculaire de 1/1, **caractérisé en ce que** le co-cristal présente un motif de diffraction des rayons X sur poudre ayant des pics [2θ] choisis parmi 7,1, 9,3, 10,2, 10,7, 13,6, 13,9, 14,1, 15,5, 16,1, 16,2, 16,8, 17,5, 18,0, 19,0, 19,5, 19,9, 20,5, 21,2, 21,3, 21,4, 21,8, 22,1, 22,6, 22,7, 23,6, 24,1, 24,4, 25,2, 26,1, 26,6, 26,8, 27,4, 27,9, 28,1, 29,1, 29,9, 30,1, 31,1, 31,3, 31,7, 32,5, 32,8, 34,4, 35,0, 35,8, 36,2 et 37,2 [°], les valeurs de 2θ étant obtenues par utilisation du rayonnement du cuivre (Cu_{Kα1} 1,54060 Å).

8. Co-cristal pour une utilisation selon l'une quelconque des revendications 1 à 7, comprenant du (rac)-tramadol•HCl et du célécoxib en un rapport moléculaire de 1/1, **caractérisé en ce que** le co-cristal présente un motif infrarouge à transformée de Fourier ayant des bandes d'absorption à 3481,6 (m), 3133,5 (m), 2923,0 (m), 2667,7 (m), 1596,0 (m), 1472,4 (m), 1458,0 (m), 1335,1 (m), 1288,7 (m), 1271,8 (m), 1168,7 (s), 1237,3 (m), 1168,7 (s), 1122,6 (s), 1100,9 (m), 1042,2 (m), 976,8 (m), 844,6 (m), 820,1 (m), 786,5 (m), 625,9 (m) cm⁻¹.

9. Co-cristal pour une utilisation selon l'une quelconque des revendications 1 à 8, comprenant du (rac)-tramadol•HCl et du célécoxib en un rapport moléculaire de 1/1, **caractérisé en ce que** le co-cristal a une cellule unitaire orthorhombique ayant les dimensions suivantes :
a = 11,0323(7) Å
b = 18,1095(12) Å
c = 17,3206(12) Å.

10. Co-cristal pour une utilisation selon l'une quelconque des revendications 1 à 9, comprenant du (rac)-tramadol•HCl et du célécoxib en un rapport moléculaire de 1/1, **caractérisé en ce que**, pour le co-cristal, le pic endothermique net correspondant au point de fusion apparaît à 164 °C.

11. Co-cristal pour une utilisation selon l'une quelconque des revendications 1 à 10, dans le traitement d'une douleur aiguë, d'une douleur chronique, d'une douleur neuropathique, d'une douleur nociceptive, d'une douleur légère et sévère à modérée, d'une hyperalgésie, d'une douleur associée à une sensibilisation centrale, d'une allodynie ou d'une douleur de cancer, y compris une neuropathie diabétique ou une neuropathie périphérique diabétique et une arthrose, une fibromyalgie ; une polyarthrite rhumatoïde, une spondylarthrite ankylosante, une capsulite rétractile, ou une sciatique.

12. Co-cristal pour une utilisation selon l'une quelconque des revendications 1 à 11, dans lequel la douleur est une douleur aiguë et chronique modérée à sévère, une douleur aiguë modérée à sévère, une douleur aiguë modérée, une douleur aiguë sévère, une douleur chronique modérée à sévère, une douleur chronique modérée, ou une douleur chronique sévère.

13. Co-cristal pour une utilisation selon l'une quelconque des revendications 1 à 12, dans lequel le co-cristal est compris dans une composition pharmaceutique comprenant aussi au moins un polymère amplificateur de solubilité ; dans lequel le polymère amplificateur de solubilité est choisi parmi les copolymères greffés de polyvinylcaprolactame-poly(acétate de vinyle)-polyéthylèneglycol ou parmi la copovidone, la povidone, la cyclodextrine, le polyéthylèneglycol et les glycérides EP de lauroylmacrogol-32, de préférence dans lequel le polymère amplificateur de solubilité est choisi parmi les copolymères greffés de polyvinylcaprolactame-poly(acétate de vinyle)-polyéthylèneglycol ou d'autres polymères hydrophiles choisis parmi la copovidone, ou la povidone, mieux encore, dans lequel le polymère amplificateur de solubilité est la copovidone.
